(19) Europäisches Patentamt
European Patent Office
Office européen des brevets

(11) **EP 4 527 902 A1**

(12) **EUROPEAN PATENT APPLICATION**

(43) Date of publication:
**26.03.2025 Bulletin 2025/13**

(21) Application number: **23306541.6**

(22) Date of filing: **19.09.2023**

(51) International Patent Classification (IPC):
**C09J 153/02** (2006.01)     **C09J 191/00** (2006.01)
**C09J 123/26** (2006.01)     **C09J 7/38** (2018.01)
**A61L 15/58** (2006.01)

(52) Cooperative Patent Classification (CPC):
(C-Sets available)
**C09J 153/02; C09J 191/00;** A61L 15/585;
C08L 2205/025; C08L 2205/03; C08L 2205/035
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Bostik SA**
**92700 Colombes (FR)**

(72) Inventors:
• **ZENG, Di**
**Shanghai (CN)**
• **ZHANG, Jingying**
**Shanghai (CN)**
• **LI, Danfeng**
**Shanghai (CN)**

(74) Representative: **Arkema Patent**
**Arkema France**
**DRD-DPI**
**420, rue d'Estienne d'Orves**
**92705 Colombes Cedex (FR)**

(54)     **HOT-MELT PRESSURE SENSITIVE ADHESIVE COMPOSITION**

(57)     The invention relates to a hot-melt pressure sensitive adhesive composition comprising based on the total weight of the composition:
(a) 10 to 40 wt% of at least one styrenic block copolymer,
(b) 10 to 40 wt% of at least one liquid plasticizer,
(c) 30 to 60 wt% of at least one solid tackifying resin, and
(d) optionally one or more additives,
wherein the liquid plasticizer comprises at least one liquid petroleum resin having a dicyclopentadiene (DCPD) level of at least 5, preferably of at least 10.

Furthermore, the invention relates to an article comprising the hot-melt pressure sensitive adhesive composition according to the invention.

Finally, the invention relates to the use of the hot-melt pressure sensitive adhesive composition according to the invention.

EP 4 527 902 A1

(52) Cooperative Patent Classification (CPC): (Cont.)

C-Sets
**C09J 191/00, C08L 53/02, C08L 53/02,
C08L 23/26**

**Description**

TECHNICAL FIELD

The present invention relates to a hot-melt pressure sensitive adhesive composition and its use, and an article.

TECHNICAL BACKGROUND

Hot-melt compositions (HM compositions) are substances which are solid at room temperature and which generally comprise neither water nor solvent. They are typically used as adhesives.

Among these HM compositions, hot-melt pressure sensitive adhesives (HMPSA) are substances conferring an immediate tacky property (also called "tack") to a substrate coated with such adhesive which allows its instantaneous adhesion to another substrate under a slight and brief pressure, at room temperature (e.g. between 18°C and 25°C).

HM adhesives are generally applied in the molten state after heating to a temperature most often comprised between 130°C and 180°C, and solidify during cooling. HM adhesives not being HMPSA thus form a seal (or adhesive joint) after cooling which ensures the attachment of the substrates to be assembled, whereas HMPSA bond substrates under a slight and brief pressure at room temperature.

HM adhesives are widely used in various applications, in particular in tapes and labels, packaging and hygiene products, such as disposable absorbent articles.

The present invention aims at providing a HMPSA composition having a high peel strength. The present invention also aims at providing a HMPSA composition having a high tackiness and/or cohesion strength.

It has now been found that one or more of these aims can be achieved by the composition described below.

SUMMARY OF THE INVENTION

The invention relates to a hot-melt pressure sensitive adhesive composition comprising based on the total weight of the composition:

(a) 10 to 40 wt% of at least one styrenic block copolymer,
(b) 10 to 40 wt% of at least one liquid plasticizer,
(c) 30 to 60 wt% of at least one solid tackifying resin, and
(d) optionally one or more additives,

wherein the liquid plasticizer comprises at least one liquid petroleum resin having a dicyclopentadiene (DCPD) level of at least 5, preferably of at least 10.

Furthermore, the invention relates to an article comprising at least one surface coated with the hot-melt pressure sensitive adhesive composition according to the invention.

Finally, the invention relates to the use of the hot-melt pressure sensitive adhesive composition according to the invention for coating a surface.

The present invention makes it possible to achieve one or more of the aims mentioned above. In particular, the composition according to the invention surprisingly has a high peel strength, especially compared to compositions devoid of the claimed liquid petroleum resin.

DESCRIPTION OF THE INVENTION

**Hot melt pressure sensitive adhesive composition**

The term "hot melt" is used herein to describe that the adhesive composition is solid at ambient temperature (e.g. 18°C to 25°C) and requires to be heated to melt and then be applied on a substrate. The adhesive composition according to the invention is generally in a molten state at a temperature of at least 115°C, preferably at least 130°C.

Liquid plasticizer

The composition according to the invention comprises 10 to 40 wt%, preferably 25 to 35 wt%, of at least one liquid plasticizer based on the total weight of the composition, the liquid plasticizer comprising at least one liquid petroleum resin.

In the context of the invention, the ranges of values are understood to be inclusive. For example, the range "10 to 40 wt%" includes, in particular, the values 10 wt% and 40 wt%.

By "liquid plasticizer" and "liquid petroleum resin", it is intended a plasticizer and a petroleum resin being liquid at 23°C and at atmospheric pressure (e.g. 101325 Pa).

The liquid petroleum resin has a DCPD level of at least 5, preferably of at least 10. For example, the liquid petroleum resin may have a DCPD level comprised between 5 and 30, preferably between 10 and 20, e.g., 5, 6, 7, 8, 9, 10, 11, 12, 13, 14, 15, 16, 17, 18, 19, 20, 21, 22, 23, 24, 25, 26, 27, 28, 29, 30.

The DCPD level may be determined by quantitative C13-NMR (nuclear magnetic resonance) as follows:

$$\text{DCPD level} = \frac{Signal\ intensity\ between\ 25-70\ ppm}{Signal\ intensity\ between\ 0-24 ppm}$$

In particular, the DCPD level may be determined according to the test method defined in the example part below.

Advantageously, the liquid petroleum resin has a hydrogenation level of less than 1, preferably less than 0.6, more preferably less than 0.4. For example, the hydrogenation level may be comprised between 0.01 and 0.9, preferably between 0.1 and 0.5, more preferably between 0.15 and 0.34, e.g., 0.9, 0.8, 0.7, 0.6, 0.5, 0.4, 0.34, 0.3, 0.2, 0.15, 0.1, 0.05, 0.01.

The hydrogenation level may be determined by gel permeation chromatography (GPC) as follows:

$$\text{Hydrogenation level} = \frac{UV\ Signal\ intensity}{RI\ Signal\ intensity}.$$

In particular, the hydrogenation level may be determined according to the test method defined in the example part below.

The color Gardner of the liquid petroleum resin may be less than 1, and/or the viscosity of the liquid petroleum resin may be 3000 to 20000 mPa.s at 30°C.

The color Gardner may be measured according to ASTM D6166-2016.

Preferably, the viscosity of the liquid petroleum resin is 6000 to 15000 mPa.s at 30°C, more preferably 8000 to 15000 mPa.s at 30°C.

The viscosity may be measured according to ASTM D3236-2021 using a Brookfield viscometer.

The liquid petroleum resin may be a reaction product resulting from the polymerization process of C5-hydrocarbon monomers, C9- hydrocarbon monomers and/or DCPD monomers.

Advantageously, the content of the at least one liquid petroleum resin in the at least one liquid plasticizer is more than 30 wt%, preferably more than 45 wt%, in particular more than 60 wt%, based on the total weight of the at least one liquid plasticizer.

The total content of the at least one liquid petroleum resin in the composition according to the invention may be comprised between 5 and 40 wt%, preferably between 10 and 35 wt%.

The at least one liquid plasticizer may further comprise a naphthenic oil, a paraffinic oil and/or an olefin oligomer, in particular a naphthenic oil.

Naphthenic and paraffinic oils are petroleum based oils which consists in a mixture of naphthenic hydrocarbons (e.g. aliphatic, saturated or unsaturated, C4 to C7-member hydrocarbon rings, preferably aliphatic, saturated or unsaturated, C4 to C6-member rings, including cycloalkanes such as cyclopentane, cyclohexane, cycloheptane), paraffinic hydrocarbons (saturated, linear or branched, alkanes) and aromatic hydrocarbons (aromatic hydrocarbon rings, which may be monocyclic or polycyclic; preferably aromatic C6-member hydrocarbon rings).

The classification of naphthenic and paraffinic oil is made based on the amount of each type of hydrocarbons in the oil. Typically, paraffinic oils have a paraffinic hydrocarbon content of at least 50% by weight with respect to the total weight of the paraffinic oil, and naphthenic oils have a naphthenic hydrocarbon content between 30% and 40% by weight, with respect to the total weight of the naphthenic oil.

By "olefin oligomers", it is intended polyolefins having a low number average molecular weight (Mn), preferably between about 100 and about 10000 g/mol. Mn can be measured by size exclusion chromatography, preferably with a polystyrene calibration.

In the present application, by "about X", it is intended more or less 10% the value of X.

As examples of olefin oligomers, mention may be made of polypropylene, polybutene, hydrogenated polyisopropene and hydrogenated polybutadiene.

Styrenic block copolymer

The composition according to the invention comprises 10 to 40 wt%, preferably 20 to 30 wt%, of at least one styrenic block copolymer based on the total weight of the composition.

By "styrenic block copolymer", it is intended a block copolymer comprising at least one polystyrene block. Preferably, the styrenic block copolymer is a linear or radial block copolymer (preferably linear) comprising at least one non elastomeric block A being a polystyrene block and at least one elastomeric block B being a totally or partially hydrogenated or a non-hydrogenated diene polymer block.

Advantageously, the at least one styrenic block copolymer is a styrene-butadiene copolymer, a styrene-isoprene copolymer, a styrene-ethylene-butylene copolymer, a styrene-ethylene-propylene copolymer, a styrene-butadiene-styrene copolymer, a styrene-isoprene-styrene copolymer, a styrene-ethylene-butylene-styrene copolymer, a styrene-butadiene-butylene-styrene copolymer, a styrene-ethylene-propylene-styrene copolymer, a styrene-ethylene-ethylene-propylene-styrene copolymer, a styrene-isoprene-butadiene-styrene copolymer or any mixture thereof.

Preferably, the at least one styrenic block copolymer is a styrene-butadiene-styrene copolymer, a styrene-isoprene-styrene copolymer, a styrene-ethylene-butylene-styrene copolymer, a styrene-butadiene-butylene-styrene copolymer, a styrene-ethylene-propylene-styrene copolymer or any mixture thereof, more preferably a styrene-butadiene-styrene copolymer, a styrene-isoprene-styrene copolymer or any mixture thereof.

The styrenic triblock copolymers may be obtained using processes known per se and are commercially available. The processes for obtaining these commercial products may result in the formation of variable amounts of diblock compounds of formula A-B. Thus, when the at least one styrenic block copolymer comprises a triblock copolymer (in particular as described above), it may further comprise between 0 % and 90 % by weight of diblock compounds, with respect to the total weight of styrenic block copolymer(s).

The at least one styrenic block copolymer may have a styrene content comprised between 10 % and 50 % by weight with respect to the total weight of the copolymer. The styrene content is the weight percentage of styrene moieties (present in the polystyrene block(s)) with respect to the total weight of the copolymer.

The styrene content can be measured by refractive index, for example according to ASTM D5775.

Unless otherwise stated, standards mentioned throughout the present application are those in effect on the date the application is filed.

The at least one styrenic block copolymer may comprise a styrene-butadiene-styrene copolymer having a styrene content of 10 to 50 wt%, preferably 20 to 30 wt%, based on the total weight of the styrene-butadiene-styrene copolymer and/or a diblock content of 50-80 wt%, preferably 68 to 80 wt%, based on the total weight of the styrene-butadiene-styrene copolymer, and/or the at least one styrenic block copolymer may comprise a styrene- isoprene-styrene copolymer having a styrene content of 20 to 35 wt%, preferably 20 to 30 wt%, based on the total weight of the styrene-isoprene-styrene copolymer, and/or a diblock content of 0-75 wt%, preferably 10 to 25 wt%, based on the total weight of the styrene-isoprene-styrene copolymer.

The diblock content can be measured by High Performance Size-Exclusion Chromatography, for example according to ASTM D5296.

The at least one styrenic block copolymer may be a combination of at least one styrene-butadiene-styrene copolymer and at least one styrene-isoprene-styrene copolymer, the weight ratio of the at least one styrene-butadiene-styrene copolymer to the at least one styrene-isoprene-styrene-copolymer being preferably 1:2 to 2:1, in particular 1:1.5 to 1.5:1.

Said styrene-butadiene-styrene copolymer and styrene-isoprene-styrene copolymer may be as described above regarding their styrene content and diblock content.

The melt index of the at least one styrenic block copolymer may be 5 to 60 g/10min, preferably 8 to 30 g/10min, at 200°C with 5 kg load.

The melt index can be measured by extrusion plastometer, for example according to ASTM D1238 (2020).

The at least one styrenic block copolymer may have a number average molecular weight (Mn) comprised between 50000 g/mol and 500000 g/mol. The number average molecular weight can be measured by size exclusion chromatography, preferably with a polystyrene calibration.

Solid tackifying resin

The composition according to the invention comprises 30 to 60 wt%, preferably 35 to 50 wt%, of at least one solid tackifying resin based on the total weight of the composition.

By "solid tackifying resin", it is intended a tackifying resin being solid at 23°C and at atmospheric pressure (e.g. 101325 Pa).

The solid tackifying resin may have a softening point of 90 to 135°C, preferably 90 to 110°C.

The softening point may be measured by a ring and ball method, for example according to ASTM E28..

The at least one solid tackifying resin may be selected from:

- natural and modified rosin, such as gum rosin, wood rosin, tall-oil rosin, distilled rosin, hydrogenated rosin, dimerized rosin, polymerized rosin,
- glycerol and pentaerythritol esters of natural and modified rosins, such as glycerol esters of pale wood rosin, glycerol esters of polymerized rosin, pentaerythritol esters of pale wood rosin, pentaerythritol esters of tall oil rosin, phenolic modified pentaerythritol esters of rosin,
- polyterpene resins, generally resulting from the polymerization of terpene hydrocarbons, such as the monoterpene known as pinene, in the presence of Friedel-Crafts catalysts preferably at moderately low temperatures (e.g. about 20°C-50°C),
- copolymers of terpene with a diene monomer, preferably an aromatic diene monomer such as a styrene monomer (e.g. styrene, methylstyrene, etc),
- phenolic-modified terpene resins, such as those resulting from the condensation, in an acidic medium, of a terpene and a phenol,
- aliphatic petroleum hydrocarbon resins (C5), resulting from the polymerization of C5-hydrocarbon monomers,
- aromatic petroleum hydrocarbon resins (C9), resulting from the polymerization of C9-hydrocarbon monomers,
- petroleum hydrocarbon resins (C5/C9), resulting from the polymerization of a blend of aliphatic C5- and aromatic C9-hydrocarbon monomers,
- dicyclopentadiene petroleum resins (DCPD), resulting from the polymerization of dicyclopentadiene monomers optionally in mixture with aromatic C9-hydrocarbon monomers and/or aliphatic C5-hydrocarbon monomers, in particular aromatic C9-hydrocarbon monomers,
- their corresponding hydrogenated derivatives (resulting from a subsequent total or partial hydrogenation thereof), and
- mixtures thereof.

As examples of C5-hydrocarbon monomers useful to prepare the aliphatic petroleum C5-hydrocarbon resin or the petroleum C5/C9-hydrocarbon resin, mention may be made of trans-1,3-pentadiene, cis-1,3-pentadiene, 2-methyl-2-butene, cyclopentadiene, methylcyclopentadiene and/or cyclopentene.

As examples of C9-hydrocarbon monomers useful to prepare the aromatic petroleum C9-hydrocarbon resin, the petroleum C5/C9-hydrocarbon resin or the DCPD resin, mention may be made of vinyltoluene, indene, methylstyrene, $\alpha$-methylstyrene, styrene and/or methylindene.

Preferably, the at least one solid tackifying resin is selected from:

- aliphatic petroleum hydrocarbon resins (C5), resulting from the polymerization of C5-hydrocarbon monomers,
- aromatic petroleum hydrocarbon resins (C9), resulting from the polymerization of C9-hydrocarbon monomers,
- petroleum hydrocarbon resins (C5/C9), resulting from the polymerization of a blend of aliphatic C5- and aromatic C9-hydrocarbon monomers,
- dicyclopentadiene petroleum resins (DCPD), resulting from the polymerization of dicyclopentadiene monomers (optionally in mixture with aromatic C9-hydrocarbon monomers and/or aliphatic C5-hydrocarbon monomers),
- their corresponding hydrogenated derivatives (resulting from a subsequent total or partial hydrogenation thereof), and
- mixtures thereof.

More preferably, the at least one solid tackifying resin is selected from:

- aromatic petroleum hydrocarbon resins (C9), resulting from the polymerization of C9-hydrocarbon monomers,
- petroleum hydrocarbon resins (C5/C9) resulting from the polymerization of a blend of aliphatic C5- and aromatic C9-hydrocarbon monomers,
- their corresponding hydrogenated derivatives (resulting from a subsequent total or partial hydrogenation thereof), and
- mixtures thereof.

According to a preferred embodiment, the at least one solid tackifying resin is hydrogenated (totally or partially).

Additives

The composition according to the invention optionally comprises one or more additives which may be selected from additional polymers other than styrenic block copolymers, ultraviolet (UV) stabilizers (or antioxidants), waxes, fillers, pigments, ultraviolet or infrared fluorescent agents, and mixtures thereof.

Advantageously, the composition according to the invention comprises one or more additives selected from UV stabilizers (or antioxidants).

The total content of additives in the composition according to the invention may be up to 20 wt% based on the total weight of the composition, preferably from 0.5 % to 2 wt%.

Examples of additional polymers other than styrenic block copolymers are polyolefins, amorphous poly-alpha-olefins, ethyl-vinyl-acetate polymers (EVA) and polyamides.

The weight average molecular weight (Mw) of said additional polymers may be comprised between 12000 g/mol and 100000 g/mol, preferably between 30000 and 80000 g/mol. Mw can be measured by size exclusion chromatography, preferably with a polystyrene calibration.

Advantageously, the composition according to the invention comprises a UV stabilizer (or antioxidant). UV stabilizers are typically introduced to protect the composition from degradation resulting from reaction with oxygen which is likely to be formed by the action of heat or light. These compounds may include antioxidants capable of scavenging free radicals.

Examples of UV stabilizers are benzotriazoles (such as 2-(2'-hydroxy-5'-methylphenyl)-5-benzotriazole), benzophenones, hindered amines (also named HALS for "hindered amine light stabilizers"), hindered phenols and multifunction phenols, such as sulfur and phosphorous-containing phenols.

Representative hindered amines include bis(1-octyloxy-2,2,6,6-tetramethyl-4-piperidyl)sebacate, bis(2,2,6,6-tetramethyl-4-piperidyl)sebacate, bis(1,2,2,6,6-pentamethyl-4-piperidyl)sebacate (CAS: 41556-26-7), methyl 1,2,2,6,6-pentamethyl-4-piperidyl sebacate (CAS: 82919-37-7) and/or 4,4'-bis($\alpha,\alpha$-dimethylbenzyl)diphenylamine.

Hindered phenols are well known to those skilled in the art and may be characterized as phenolic compounds that also contain sterically bulky radicals in close proximity to the phenolic hydroxyl group thereof. In particular, tertiary butyl groups generally are substituted onto the benzene ring in at least one of the ortho positions relative to the phenolic hydroxyl group. Representative hindered phenols and multifunction phenols include: 1,3,5-trimethyl-2,4,6-tris(3-5-di-tert-butyl-4-hydroxybenzyl) benzene, pentaerythritol tetrakis[3-(3,5-di-tert-butyl-4-hydroxyphenyl)propionate], n-octadecyl-3(3,5-di-tert-butyl-4-hydroxyphenyl) propionate, 4.4'-methylenebis(4-methyl-6-tert-butylphenol), 4,4'-thiobis(6-tert-butyl-o-cresol), 2,6-di-tert-butylphenol, 6-(4-hydroxyphenoxy)-2,4-bis(n-octylthio)-1,3,5-triazine, 2,3,6-tris(4-hydroxy-3,5-di-tert-butyl-phenoxy)-1,3,5-triazine, di-n-octadecyl-3,5-di-tert-butyl-4-hydroxybenzyl-phosphonate, 2-(n-octyl-thio)ethyl-3,5-di-tert-butyl-4-hydroxybenzoate, sorbitol hexa-3(3,5-di-tert-butyl-4-hydroxy-phenyl)propionate, 2,2'-methylene bis(4-methyl-6-tert-butylphenol)phosphites, tris-(p-nonylphenyl)-phosphite (TNPP), bis(2,4-di-tert-butylphenyl)4,4'-diphenylenediphosphonite, tris(2,4-di-tert-butylphenyl) phosphite and/or tris(2,4-di-tert-butylphenyl)phosphate.

The total UV stabilizer (or antioxidant) content may be comprised between 0.2 % and 2 wt% based on the total weight of the composition according to the invention.

Examples of waxes are paraffin waxes, Fischer-Tropsch waxes and EVA waxes.

By "paraffin wax", it is intended a wax derived from crude oil. It generally consists of a complex mixture of hydrocarbons. Paraffin waxes often contain a majority of straight-chain hydrocarbons, and can also contain branched hydrocarbons such as isoparaffins and other branched materials, and cycloalkanes such as cycloparaffins and other cyclocontaining materials. Paraffin waxes are characterized by a clearly defined crystal structure.

By "Fischer-Tropsch wax", it is intended a wax obtained by the so-called Fischer-Tropsch process. The Fischer-Tropsch process includes converting a synthesis gas comprising mainly hydrogen and carbon monoxide to hydrocarbons. The conversion is effected by contacting the synthesis gas with a Fischer-Tropsch catalyst, usually an iron or cobalt based catalyst, in a fixed bed or a slurry bed reactor under either low or high temperature Fischer-Tropsch operating conditions. In this manner, a mixture of hydrocarbons having different boiling ranges is obtained. The Fischer-Tropsch wax is then recovered, e.g. by means of distillation, from this hydrocarbon mixture. The Fischer-Tropsch wax typically has a composition wherein about 80% by volume thereof has a boiling point higher than 550°C atmospheric equivalent temperature ("AET").

By "EVA wax", it is intended oligomeric polymer compounds that are prepared via a process comprising the copolymerization of ethylene monomers and vinyl acetate monomers and that have the following properties: (a) solid at room temperature; (b) low melting point; and (c) insoluble in water. The EVA copolymers of the EVA wax may be functionalized or modified in any possible manner.

Typical fillers include talc, calcium carbonate, clay, silica, mica, wollastonite, feldspar, aluminum silicate, alumina, hydrated alumina, glass microspheres, ceramic microspheres and/or thermoplastic microspheres.

The pigment may be an organic and/or inorganic pigment, for example titanium dioxide.

Examples of ultraviolet or infrared fluorescent agents are 2-(6-hydroxy-3-oxo-(3H)-xanthen-9-yl) benzoic acid, disodium 6-hydroxy-3-oxo-9-xanthene-o-benzoate and 8-hydroxy-1,3,6-pyrenetrisulfonic acid trisodium salt.

<u>Other features of the hot melt adhesive composition according to the invention</u>

According to an embodiment, the hot-melt pressure sensitive adhesive composition according to the invention comprises, based on the total weight of the composition:

    (a) 20 to 30 wt% of at least one styrenic block copolymer,
    (b) 25 to 35 wt% of at least one liquid plasticizer,
    (c) 35 to 50 wt% of at least one solid tackifying resin, and
    (d) optionally 0.5 to 2 wt% of one or more additives.

Preferably, the composition according to the invention consists essentially of the ingredients mentioned above.

By "consisting essentially", it is intended that said composition comprises less than 5 % by weight of ingredients other than the aforementioned ingredients, relative to the total weight of said composition, preferably less than 2% by weight, even more preferably less than 1% by weight.

The ingredients of this embodiment and their particular contents are as described above, including the embodiments and preferred features.

In particular, the hot-melt pressure sensitive adhesive composition comprises (or consists essentially of), based on the total weight of the composition:

(a) 20 to 30 wt% of at least one styrenic block copolymer being a styrene-butadiene-styrene copolymer, a styrene-isoprene-styrene copolymer, a styrene-ethylene-butylene-styrene copolymer, a styrene-butadiene-butylene-styrene copolymer, a styrene-ethylene-propylene-styrene copolymer or any mixture thereof,

(b) 25 to 35 wt% of at least one liquid plasticizer comprising more than 45 wt% of at least one liquid petroleum resin based on the total weight of the at least one liquid plasticizer,

(c) 35 to 50 wt% of at least one solid tackifying resin, the solid tackifying resin being preferably selected from aliphatic petroleum C5-hydrocarbon resins, aromatic petroleum C9-hydrocarbon resins, petroleum C5/C9-hydrocarbon resins, DCPD petroleum resins, hydrogenated derivatives thereof, and mixtures thereof, and

(d) optionally 0.5 to 2 wt% of one or more additives, preferably selected from UV stabilizers (or antioxidants),

wherein the liquid petroleum resin has a hydrogenation level of less than 0.6, a DCPD level of at least 5 and a viscosity at 30°C of 6000 to 15000 mPa.s.

The softening point of the composition according to the invention may vary between 60°C and 130°C, preferably between 60°C and 110°C. The softening point can be measured by a ring and ball method, for example according to ASTM E28-2022.

The melt viscosity at 160°C of the composition according to the invention may vary between 1000 and 10000 mPa.s, preferably between 1800 and 8000 mPa.s. The melt viscosity may be measured according to ASTM D3236-2021, with a Brookfield viscometer.

Advantageously, the composition according to the invention has an improved peel strength, in particular compared to a composition lacking the liquid petroleum resin. The peel strength may be measured for a cotton/polyethylene laminated specimen, for example as described in Example 1. Said peel strength is preferably of at least 3.0 N/20 mm, more preferably at least 3.2 N/20 mm.

The composition according to the invention can be prepared by mixing its ingredients under heat, preferably at a temperature ranging from 140°C to 180°C and atmospheric pressure. An example of preparation is described in the example part below.

## Article

The invention further relates to an article comprising the hot-melt pressure sensitive adhesive composition according to the invention, wherein the article comprises at least one surface coated with the hot-melt pressure sensitive adhesive composition according to the invention.

The at least one coated surface may be a textile (woven or nonwoven), an absorbent fluff, a super absorbent polymer (SAP), a composite material and/or a plastic which may be elastomeric or non-elastomeric (such as styrene block copolymer(s), polyurethane(s) and/or polyolefin(s)).

Preferably, the surface is a textile, preferably comprising polypropylene, polyethylene, polyethylene terephthalate, cotton, bamboo, silk and/or polylactic acid.

According to a preferred embodiment, the composition according to the invention bonds at least two substrates of the article according to the invention (preferably, an assembly product). The at least two substrates may be joined adhesively by a layer of the composition according to the invention, in sandwich between the two substrates, and/or by spots of the composition according to the invention.

The article may be a disposable diaper, disposable training pants, a disposable adult incontinent pad or brief, or a disposable feminine sanitary napkin or pad.

## Use of the hot-melt pressure sensitive adhesive composition

The invention also relates to the use of the hot-melt pressure sensitive adhesive composition according to the invention for coating a surface.

The composition according to the invention is thus used to provide adhesive properties to the surface.

The surface to be coated may be a textile (woven or nonwoven), an absorbent fluff, a super absorbent polymer (SAP), a composite material and/or a plastic which may be elastomeric or non-elastomeric (such as styrene block copolymer(s), polyurethane(s) and/or polyolefin(s)).

Preferably, the surface is a textile, preferably of a part of an article selected from disposable diapers, disposable training pants, disposable adult incontinent pads or briefs and disposable feminine sanitary napkins or pads.

All the embodiments described above can be combined with each other. In particular, the various aforementioned ingredients in the composition, and in particular the preferred embodiments, can be combined with each other.

## EXAMPLES

The following examples illustrate the invention without limiting it.

Raw materials used in the examples

The components used in the examples are described in Table 1 below.

Table 1

| Raw Material | Supplier | Detailed information |
|---|---|---|
| Quintac® 3280 | ZEON corporation | Styrene-isoprene-styrene block copolymer, Styrene content of 25 wt%, Diblock content of 17 wt%, Melt index of 12 g/10min at 200°C with 5 kg load |
| DZ 4504 | Nanjing Haiqi Environmental Protection Technology Co. | Styrene-butadiene-styrene block copolymer, Styrene content of 22 wt%, Diblock content of 77 wt%, Melt index of 14-24 g/10min at 200°C with 5 kg load |
| HAISIS™ JH-8291 | Ningbo Jinhai Chenguang Chemical Corporation | Styrene-isoprene-styrene block copolymer, Styrene content of 29 wt%, Diblock content of 0 wt%, Melt index of 10.4 g/10min at 200°C with 5 kg load |
| HAISIS™ JH 9270 | Ningbo Jinhai Chenguang Chemical Corporation | Styrene-isoprene-styrene block copolymer, Styrene content of 24 wt%, Diblock content of 68 wt%, Melt index of 20 g/10min at 200°C with 5 kg load |
| Baling® YH-1209 | SINOPEC | Styrene-isoprene-styrene block copolymer, Styrene content of 29 wt%, Diblock content of less than 1 wt%, Melt index of 6-15 g/10min at 200°C with 5 kg load |
| GLOBALPRENE 5526 | LCY GRIT CORP. | Styrene-isoprene-styrene block copolymer, Styrene content of 25 wt% detected by ASTM D5775, Diblock content of 25 wt% detected by ASTM D5296, Melt index of 6-15 g/10min at 190°C with 5 kg load, detected by ASTM D 1238 |
| GLOBALPRENE 3545 | LCY GRIT CORP. | Styrene-butadiene-styrene block copolymer, Styrene content of 45 wt% detected by ASTM D5775, Diblock content of 63 wt% detected by ASTM D5296, Melt index of 55 g/10min at 190°C with 5 kg load, detected by ASTM D 1238 |

(continued)

| Raw Material | Supplier | Detailed information |
|---|---|---|
| HAITACK™ JH-6130 | Ningbo Jinhai Chenguang Chemical Corporation | Solid tackifying resin, cycloaliphatic hydrocarbon resin, Softening Point of 131°C, Melt Viscosity of 2400 mPa.s at 180°C |
| HA-100 | Henghe Materials & Science Technology Co., Ltd. | Solid tackifying resin, C9 hydrogenated hydrocarbon resin, Softening Point of 99.5°C Melt Viscosity of 576 mPa.s at 160°C |
| HM-1000 | Henghe Materials & Science Technology Co., Ltd. | Solid tackifying resin, C9 hydrogenated hydrocarbon resin, Softening Point of 103°C Melt Viscosity of 141 mPa.s at 160°C |
| Irganox® 1010 | BASF | Antioxidant, Pentaerythritol tetrakis(3-(3,5-di-tert-butyl-4-hydroxyphenyl)propionate) |
| EVERFOS®-168 | Everspring Chemical Co., Ltd. | Antioxidant, Tris (2,4-di-butylphenyl) phosphite |
| RIASORB UV-P | Rianlon Corporation | Light stabilizer, 2-(2'-Hydroxy-5'-methylphenyl)-5-benzotriazole |
| N4010 | PetroChina Karamay Petrochemical Company | Liquid plasticizer, Naphthenic Base Mineral Oil |
| PB450 | Daelim Industrial Co., Ltd. | Polyisobutylene, liquid, Mn=450 g/mol |
| HM-10 | Henghe Materials & Science Technology Co., Ltd. | Hydrogenated liquid petroleum oil, DCPD level =13.8, hydrogenation level=0.27 Color Gardner <1, water-white, Viscosity: 10,500 mPa.s at 30°C |
| Regalite™ C8010 | Eastman | Hydrogenated liquid petroleum oil, DCPD level =3.7, hydrogenation level=0.63 Color Gardner <1, water-white |

Testing method

The following measurement methods are used:
Color Gardner: measured according to ASTM D6166-2016.
Viscosity/Melt viscosity: measured according to ASTM D3236-2021 using Brookfield viscometer.

DCPD level:

The sample is tested by quantitative C13-NMR (600MHz Avance III). In particular, the sample was prepared at room temperature (about 25°C) with deuterated chloroform as the solvent and the probe was set at 25°C. DCPD level is defined as follows:

$$DCPD\ level = \frac{Signal\ intensity\ between\ 25-70\ ppm}{Signal\ intensity\ between\ 0-24 ppm}$$

Hydrogenation level:

The sample is tested by GPC, the UV (ultraviolet) signal and RI (refractive index) signal are collected separately. In particular, the testing was performed on Waters Alliance 2698 instrument with RI and UV-254nm detectors. Polystyrene with different molecular weights were used to build the calibration curve. About 0.1g of sample was dissolved in 10mL tetrahydrofuran and 0.1mL toluene. The solution was filtered and 50μL was injected into the instrument for analysis.

The hydrogenation level is then defined as follows:

$$\text{Hydrogenation level} = \frac{UV\ Signal\ intensity}{RI\ Signal\ intensity}$$

Softening point: the Ring and Ball softening point, measured by ASTM E28-2022 with an automated Herzog unit.

Peal strength:

Laminates are prepared through a continuous coating process in a hot melt coater for lab use produced by Shanghai Huadi Machinery Co., LTD by: coating the adhesives composition to be tested at a temperature of about 160°C, with the primary and second substrates being release film and polyethylene (PE) film, at a quantity of 30g/m$^2$, using a slot nozzle. After their production, these laminates are packaged by being wound up. After being held at room temperature overnight, the peel strength of these laminates is then assessed as follows: a sample of said laminate is cut to a length of about 150mm and a width of about 50mm, after the release film is peeled off, the remaining PE film with said adhesive is attached to commercially available cotton fabric, and then rolled by a 2kg weight back and force once. The as prepared sample is then peeled by tensile machine (from Electrochemical Universal Testing Machine, Model CMT2102) with a speed 300mm/min. Five samples are tested and then averaged and recorded.

The adhesive compositions of Examples according to the invention (E1-E6) and Comparative Examples (CE1-CE4) are prepared as follows with the amounts of the raw materials in weight percentages listed in Table 2:

All the ingredients except the solid tackifying resins were added into the reactor. The reactor was heated to 160°C, and stirring was performed for 1 to 2 hours. The solid tackifying resins were then added and stirring was performed for 1 h to obtain the adhesive composition.

Table 2

| | E1 | E2 | E3 | E4 | E5 | E6 | CE 1 | CE 2 | CE 3 | CE 4 |
|---|---|---|---|---|---|---|---|---|---|---|
| N4010 | | | | | | 14 | | | | 28 |
| PB450 | | | | | | | | | 32 | |
| HM-10 | 33 | 33 | 33 | 33 | 32 | 14 | | | | |
| Regalite™ C8010 | | | | | | | 32 | 32 | | |
| Quintac® 3280 | 12 | 12 | 12 | 12 | 12 | 10 | 12 | 12 | 12 | 12 |
| DZ 4504 | 12 | | | | | 15 | | 13 | 13 | |
| HAISIS™ JH-8291 | | | | | | | | | | 11 |
| HAISIS™ JH 9270 | | 12 | | | | | 13 | | | |
| Baling® YH-1209 | | | 12 | | | | | | | |
| GLOBALPRENE 5526 | | | | 12 | | | | | | |
| GLOBALPRENE 3545 | | | | | 15 | | | | | |
| HAITACK™ JH-6130 | | | | | | | | | 2 | 8 |
| HA-100 | 10 | 10 | 10 | 10 | 10 | 14 | 10 | 10 | 10 | 10 |
| HM-1000 | 32 | 32 | 32 | 32 | 30 | 32 | 32 | 32 | 30 | 30 |
| Irganox® 1010 | 0.5 | 0.5 | 0.5 | 0.5 | 0.5 | 0.5 | 0.5 | 0.5 | 0.5 | 0.5 |
| EVERFOS®-168 | 0.2 | 0.2 | 0.2 | 0.2 | 0.2 | 0.2 | 0.2 | 0.2 | 0.2 | 0.2 |
| RIASORB UV-P | 0.3 | 0.3 | 0.3 | 0.3 | 0.3 | 0.3 | 0.3 | 0.3 | 0.3 | 0.3 |

The testing results are shown in Table 3.

Table 3

| | E1 | E2 | E3 | E4 | E5 | E6 | CE1 | CE2 | CE3 | CE4 |
|---|---|---|---|---|---|---|---|---|---|---|
| Softening point / °C | 66 | 73.2 | 75.2 | 73 | 75.8 | 70.1 | 93.4 | 87.7 | 83.9 | 89 |
| Melt Viscosity at 160°C / mPa.s | 3125 | 2255 | 2500 | 2490 | 2505 | 4080 | 2730 | 3988 | 3725 | 1835 |
| Peel strength, N/20mm | 4.14 | 3.40 | 3.52 | 3.50 | 3.37 | 3.85 | 2.43 | 2.91 | 1.96 | 2.2 |

As is shown in the Examples, the adhesive composition with the liquid petroleum resin as claimed has better peel strength, suitable viscosity and softening point. For the Comparative Examples, with the inadequate liquid plasticizer, they have a lower peel strength.

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

**Claims**

1. A hot-melt pressure sensitive adhesive composition comprising based on the total weight of the composition:

   (a) 10 to 40 wt% of at least one styrenic block copolymer,
   (b) 10 to 40 wt% of at least one liquid plasticizer,
   (c) 30 to 60 wt% of at least one solid tackifying resin, and
   (d) optionally one or more additives,

   wherein the liquid plasticizer comprises at least one liquid petroleum resin having a dicyclopentadiene (DCPD) level of at least 5, preferably of at least 10.

2. The hot-melt pressure sensitive adhesive composition according to claim 1, wherein the liquid petroleum resin has a hydrogenation level of less than 1, preferably less than 0.6, more preferably less than 0.4.

3. The hot-melt pressure sensitive adhesive composition according to any one of claims 1 to 2, wherein the color Gardner of the liquid petroleum resin is less than 1, and/or the viscosity of the liquid petroleum resin is 3000 to 20000 mPa.s at 30°C.

4. The hot-melt pressure sensitive adhesive composition according to any one of claims 1 to 3, wherein the content of the at least one liquid petroleum resin in the at least one liquid plasticizer is more than 30 wt%, preferably more than 45 wt%, in particular more than 60 wt%, based on the total weight of the at least one liquid plasticizer.

5. The hot-melt pressure sensitive adhesive composition according to any one of claims 1 to 4, wherein the at least one styrenic block copolymer is a styrene-butadiene copolymer, a styrene-isoprene copolymer, a styrene-ethylene-butylene copolymer, a styrene-ethylene-propylene copolymer, a styrene-butadiene-styrene copolymer, a styrene-isoprene-styrene copolymer, a styrene-ethylene-butylene-styrene copolymer, a styrene-butadiene-butylene-styrene copolymer, a styrene-ethylene-propylene-styrene copolymer, a styrene-ethylene-ethylene-propylene-styrene copolymer, a styrene-isoprene-butadiene-styrene copolymer or any mixture thereof.

6. The hot-melt pressure sensitive adhesive composition according to claim 5, wherein the at least one styrenic block copolymer comprises a styrene-butadiene-styrene copolymer having a styrene content of 10 to 50 wt%, preferably 20 to 30 wt%, based on the total weight of the styrene-butadiene-styrene copolymer and/or a diblock content of 50-80 wt%, preferably 68 to 80 wt%, based on the total weight of the styrene-butadiene-styrene copolymer, and/or wherein the at least one styrenic block copolymer comprises a styrene- isoprene-styrene copolymer having a styrene content of 20 to 35 wt%, preferably 20 to 30 wt%, based on the total weight of the styrene-isoprene-styrene copolymer, and/or a diblock content of 0-75 wt%, preferably 10 to 25 wt%, based on the total weight of the styrene-isoprene-styrene copolymer.

7. The hot-melt pressure sensitive adhesive composition according to any one of claims 5 to 6, wherein the at least one styrenic block copolymer is a combination of at least one styrene-butadiene-styrene copolymer and at least one styrene-isoprene-styrene copolymer, the weight ratio of the at least one styrene-butadiene-styrene copolymer to the at least one styrene-isoprene-styrene-copolymer being 1:2 to 2:1.

8. The hot-melt pressure sensitive adhesive composition according to any one of claims 1 to 7, wherein the melt index of the at least one styrenic block copolymer is 5 to 60 g/10min, preferably 8 to 30 g/10min, at 200°C with 5 kg load.

9. The hot-melt pressure sensitive adhesive composition according to any one of claims 1 to 8, wherein the solid tackifying resin has a softening point of 90 to 135°C, preferably 90 to 110°C.

10. The hot-melt pressure sensitive adhesive composition according to any one of claims 1 to 9, wherein based on the total weight of the composition, the hot-melt pressure sensitive adhesive composition comprises:

    (a) 20 to 30 wt% of at least one styrenic block copolymer,
    (b) 25 to 35 wt% of at least one liquid plasticizer,
    (c) 35 to 50 wt% of at least one solid tackifying resin, and
    (d) optionally 0.5 to 2 wt% of one or more additives.

11. An article comprising at least one surface coated with the hot-melt pressure sensitive adhesive composition of any one of claims 1 to 10.

12. The article of claim 11, wherein the surface is a textile, preferably comprising polypropylene, polyethylene, polyethylene terephthalate, cotton, bamboo, silk and/or polylactic acid.

13. The article of claim 11 or 12, wherein the article is a disposable diaper, disposable training pants, a disposable adult incontinent pad or brief, or a disposable feminine sanitary napkin or pad.

14. Use of the hot-melt pressure sensitive adhesive composition of any one of claims 1 to 13 for coating a surface.

15. The use of claim 14, wherein the surface is a textile, preferably of a part of an article selected from disposable diapers, disposable training pants, disposable adult incontinent pads or briefs and disposable feminine sanitary napkins or pads.

Europäisches Patentamt
European Patent Office
Office européen des brevets

# EUROPEAN SEARCH REPORT

**Application Number**

EP 23 30 6541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 760 684 A1 (BOSTIK SA [FR]) 6 January 2021 (2021-01-06) * claims; examples * | 1-15 | INV. C09J153/02 C09J191/00 C09J123/26 C09J7/38 |
| A | WO 2022/043382 A1 (BOSTIK SA [FR]) 3 March 2022 (2022-03-03) * claims; examples * | 1-15 | ADD. A61L15/58 |
| A | WO 2023/102408 A1 (FULLER H B CO [US]) 8 June 2023 (2023-06-08) * claims; examples * | 1-15 | |
| A | CN 116 083 011 A (SHANGHAI YONGGUAN ZHONGCHENG NEW MATERIAL TECH GROUP CO LTD) 9 May 2023 (2023-05-09) * claims; examples * | 1-15 | |

**TECHNICAL FIELDS SEARCHED (IPC)**

C09J
C08L

The present search report has been drawn up for all claims

| Place of search | Date of completion of the search | Examiner |
|---|---|---|
| Munich | 19 February 2024 | Friebe, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 6541

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2024

| Patent document cited in search report | | Publication date | Patent family member(s) | | Publication date |
|---|---|---|---|---|---|
| EP 3760684 | A1 | 06-01-2021 | BR | 112021024906 A2 | 18-01-2022 |
| | | | CA | 3140831 A1 | 14-01-2021 |
| | | | CN | 114008160 A | 01-02-2022 |
| | | | EP | 3760684 A1 | 06-01-2021 |
| | | | EP | 3994220 A1 | 11-05-2022 |
| | | | JP | 2022538677 A | 05-09-2022 |
| | | | KR | 20220029674 A | 08-03-2022 |
| | | | US | 2022325149 A1 | 13-10-2022 |
| | | | WO | 2021004870 A1 | 14-01-2021 |
| WO 2022043382 | A1 | 03-03-2022 | CN | 115968277 A | 14-04-2023 |
| | | | EP | 4204508 A1 | 05-07-2023 |
| | | | JP | 2023538766 A | 11-09-2023 |
| | | | US | 2023323167 A1 | 12-10-2023 |
| | | | WO | 2022043382 A1 | 03-03-2022 |
| WO 2023102408 | A1 | 08-06-2023 | NONE | | |
| CN 116083011 | A | 09-05-2023 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 41556-26-7

- *CHEMICAL ABSTRACTS*, 82919-37-7